(19) Europäisches Patentamt
European Patent Office
Office européen des brevets

(11) EP 0 745 373 B1

(12) **FASCICULE DE BREVET EUROPEEN**

(45) Date de publication et mention de la délivrance du brevet:
**05.11.1997 Bulletin 1997/45**

(51) Int. Cl.$^6$: **A61K 7/06**, A61K 7/48

(21) Numéro de dépôt: **96400722.3**

(22) Date de dépôt: **03.04.1996**

(54) **Composition sous forme de mousse aérosol à base de polyuréthanne et de polymère anionique**

Aerosolschaumzusammensetzung auf der Basis von Polyurethan mit anionische Polymer

Aerosol foam composition based on polyurethane and an anionic polymer

(84) Etats contractants désignés:
**DE ES FR GB IT**

(30) Priorité: **12.05.1995 FR 9505676**

(43) Date de publication de la demande:
**04.12.1996 Bulletin 1996/49**

(73) Titulaire: **L'OREAL**
**75008 Paris (FR)**

(72) Inventeurs:
- **Dupuis, Christine**
  **75018 Paris (FR)**
- **Dubief, Claude**
  **78150 Le Chesnay (FR)**

(74) Mandataire:
**Tezier Herman, Béatrice**
**L'OREAL,**
**Département Propriété Industrielle,**
**90, rue du Gal Roguet**
**92583 Clichy Cédex (FR)**

(56) Documents cités:
**EP-A- 0 555 155** **EP-A- 0 636 361**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 745 373 B1

Printed by Rank Xerox (UK) Business Services
2.14.23/3.4

## Description

La présente invention est relative à une composition cosmétique pressurisée en aérosol en présence d'un agent propulseur et susceptible de former une mousse comprenant, dans un milieu aqueux cosmétiquement acceptable, au moins un polyuréthanne associatif et au moins un polymère anionique. Elle a également trait à l'utilisation d'un polyuréthanne associatif pour améliorer les propriétés des mousses à base de polymère anionique et d'agent propulseur.

Des compositions cosmétiques pressurisées dans des dispositifs aérosol, dans des conditions telles à former une mousse à la sortie du dispositif, sont bien connues et sont utilisées notamment dans le traitement des cheveux et/ou de la peau.
On appellera de telles compositions dans la suite de la description "mousse aérosol".

Ces mousses permettent généralement d'obtenir sur les cheveux une bonne répartition des compositions cosmétiques et elles sont en outre d'une utilisation aisée et plus économique quant à la quantité de produit utilisé par rapport aux lotions.

Ces mousses doivent être suffisamment stables pour ne pas se liquéfier rapidement et doivent également disparaître rapidement soit spontanément, soit lors du massage servant à faire pénétrer et/ou à répartir la composition sur les matières kératiniques et plus particulièrement la chevelure et/ou les cheveux.

Les mousses de coiffage et/ou de maintien des cheveux contiennent en général au moins un polymère de préférence anionique, non ionique ou amphotère qui apporte des propriétés de fixation aux cheveux.

Ces polymères sont généralement non moussants ou faiblement moussants et pour obtenir une mousse aérosol il faut donc ajouter un agent moussant et/ou un agent améliorant la qualité de la mousse.

Les agents moussants et/ou les agents améliorant la qualité de la mousse habituellement utilisés sont par exemple des agents tensioactifs anioniques, non ioniques ou amphotères; mais utilisés seuls en association avec le polymère, ces agents tensioactifs produisent des mousses aérosol de qualité non satisfaisante. En effet, soit elles se liquéfient au moment de l'application, soit elles ne disparaissent pas après l'application même lors du massage. De plus, il se produit un phénomène de remoussage sur cheveux mouillés.

On a déjà proposé dans le brevet français 2505348 d'associer les polymères anioniques à des polymères cationique, l'un des deux polymères présentant des propriétés moussantes.

On a également proposé dans le brevet français 2598613 d'utiliser un alcool polyvinylique comme agent moussant dans les compositions cosmétiques.

La demanderesse a maintenant découvert que l'on pouvait améliorer les propriétés des mousses aérosol à base de polymères anioniques en ajoutant un polyuréthanne associatif. En particulier, la rigidité, l'expansion et la stabilité de la mousse sont nettement améliorées.

La présente invention a donc pour objet une composition cosmétique pressurisée en aérosol en présence d'un agent propulseur et susceptible de former une mousse, caractérisée par le fait qu'elle comprend, dans un milieu aqueux cosmétiquement acceptable, au moins un polyuréthanne associatif lequel contient au moins une séquence hydrophile, au moins une séquence hydrophobe et au moins un groupement uréthanne et au moins un polymère anionique, l'un au moins des deux polymères étant un polymère à pouvoir moussant.

L'invention a également pour objet une composition cosmétique sous forme de mousse, caractérisée par le fait qu'elle résulte de l'expansion à l'air d'une composition telle que définie ci-dessus.

L'invention concerne l'utilisation d'un polyuréthanne associatif lequel contient au moins une séquence hydrophile, au moins une séquence hydrophobe et au moins un groupement uréthanne pour améliorer les propriétés des mousses résultant de l'expansion à l'air d'une composition pressurisée en aérosol à base de polymère anionique et d'agent propulseur, l'un au moins des deux polymères étant un polymère à pouvoir moussant.

De plus, l'addition du polyuréthanne améliore les propriétés cosmétiques des compositions pressurisées en aérosol contenant un polymère anionique. En effet, les cheveux traités avec les compositions selon l'invention sont plus doux et le toucher de ces derniers est plus naturel et plus agréable. D'autre part, l'effet renforçateur des compositions est plus important.

Par ailleurs, les mousses sont agréables et faciles à appliquer sur les matières kératiniques telles que les cheveux ou la peau.

On entend par polymère à pouvoir moussant conforme à l'invention, un polymère qui en solution dans l'eau à 0,5% en poids donne selon le test de Ross Miles (norme AFNOR T 73 404) modifié en température et effectué à 20°C, une hauteur de mousse supérieure à 1 cm et après pressurisation de la solution une quantité de mousse telle que la masse volumique soit inférieure à 0,4 et de préférence inférieure à 0,25 g/cm3.
Ces tests sont décrits dans le brevet français 2505348.

De préférence, le polymère à pouvoir moussant est le polyuréthanne associatif et encore plus particulièrement, les deux polymères sont des polymères à pouvoir moussant.

Les polyuréthannes associatifs sont ici définis comme des polymères contenant au moins une séquence hydrophile, au moins une séquence hydrophobe et au moins un groupement uréthanne.

La séquence hydrophile est de préférence une séquence polyoxyalkylénée et en particulier polyoxyéthylénée.

La séquence hydrophobe peut être une chaîne grasse comprenant de préférence de 8 à 30 atomes de carbone.

Le polyuréthanne associatif comporte de préférence au moins deux séquences hydrophobes.

Selon l'invention, les polyuréthannes associatifs ont généralement un poids moléculaire compris entre 500 et 5.000.000.

Parmi les polyuréthannes associatifs utilisables selon l'invention, on peut citer les polyuréthannes appartenant à l'un des trois groupes suivants :

Groupe I

Les polyuréthannes répondant à la formule (I) suivante :

$$X\text{-}B_p\text{-}E_q\text{-}(B\text{-}E)_n\text{-}B_r\text{-}E_t\text{-}X \qquad (I)$$

dans laquelle,

n est un nombre compris entre 1 et 10, p, q, r et t, identiques ou différents, valent 0 ou 1, avec au moins q ou r valant 1 et, t valant 0 lorsque r est égal à 0,

sous réserve que:

lorsque q vaut 1, alors :

a) p = r = t = 0 , ou
b) p = 0 et r = t = 1 , ou
c) t = 0 et r = p = 1 , et

lorsque q vaut 0, alors :

r = 1 et p = t = 0 ;

Groupe II

Les polyuréthannes répondant à la formule (II) suivante :

$$(H\text{-}E\text{-}OCH_2)_sL[Q_v\text{-}(D_u\text{-}E\text{-}X)_w\text{-}R_z]_m \qquad (II)$$

dans laquelle,

m est un nombre entier variant de 2 à 4 et s un nombre entier variant de 0 à 2, la somme de m et s variant de 2 à 4, w est un nombre entier variant de 1 à 3, et chacun de u, v et z est, indépendamment l'un de l'autre, 0 ou 1 ; L représente Y, Z ou -O-, Y étant un radical hydrocarboné hydrophobe contenant au moins un atome de carbone et de préférence de 1 à 4 atomes de carbone, et Z un radical hydrophobe trivalent choisi parmi les groupements suivants :
$-OCONH(CH_2)_6N[CONH(CH_2)_6NHCOO-]_2$,
$CH_3C[CH_2O\text{-}OCNHC_7H_6NHCO-]_3$, et
$CH_3CH_2C[CH_2O\text{-}OCNHC_7H_6NHCO-]_3$
Q représente le groupe -CH2O-, et D le groupe -CH2O-, sous réserve que,

a) lorsque L représente Y, alors u et w valent chacun 1, v et z valent 0, m vaut au moins 2 et la somme de m et s est 4 ;
b) lorsque L vaut Z, alors u, v et s valent chacun 0, m vaut 3, w vaut 2 ou 3 et z est 0 ou 1 ; et
c) lorsque L représente -O-, alors v et u valent chacun 1, w varie de 1 à 3, m vaut 2, et s et z valent chacun 0 ;

dans chacune des formules de ces deux groupes, X et R représentent un radical hydrophobe; B un groupe divalent hydrophobe de formule -CONH-G-NHCO-O-, dans laquelle G est un radical divalent dérivé d'un di- ou d'un triisocyanate organique dont tous les groupes isocyanate ont réagi; et E représente un groupe polyéther divalent non ionique.

Groupe III

Les polyuréthannes de ce groupe sont obtenus par réaction (a) d'un réactif polyfonctionnel choisi parmi les polyols organiques possédant au moins trois groupements hydroxyle, les polyisocyanates organiques possédant au moins trois groupements isocyanates, et leur mélange (b) un réactif difonctionnel choisi parmi les diols organiques, les diisocyanates organiques, et leur mélange, le diol étant présent dans le mélange de la réaction lorsque le polyisocyanate est présent et le diisocyanate étant présent lorsque le polyol est présent ; (c) un composé monofonctionnel hydroxyl ou amino en une quantité suffisante pour piéger tout groupement isocyanate n'ayant pas réagi lors de la réaction entre (a) et (b), et pour prévenir la coagulation du mélange réactionnel ; et éventuellement (d) un monoisocyanate organique pour piéger les groupements hydroxyl restant après la réaction entre (a) et (b) ; réaction dans laquelle, au moins l'un du polyol et du diol contient au moins un segment polyéther soluble dans l'eau d'un poids moléculaire d'au moins 1500, la somme des atomes de carbone dans les réactifs contenant des groupements isocyanate, des groupements hydroxyl et des groupements amino, est d'au moins 20, et le poids moléculaire moyen de ces polyuréthannes est d'environ 10 000 à 200 000.

De tels polyuréthannes associatifs sont par exemple décrits dans les brevets suivants US 4179028, US 4155892, US 4327008, US 4337184, US 4373083, US 4499233, US4426485.

On peut également utiliser les polyuréthannes répondant à la formule (III) suivante:

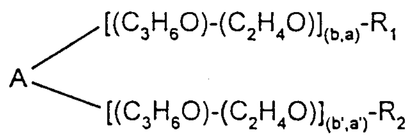

(III)

dans laquelle,

- $[(C_3H_6O)-(C_2H_4O)]_{(bi,ai)}$, avec (bi,ai), désignant (b,a) ou (b',a'), signifie qu'il s'agit d'un polymère statistique d'oxyde de propylène et d'oxyde d'éthylène contenant a moles d'oxyde d'éthylène et b moles d'oxyde de propylène réparties de manière aléatoire dans la chaîne polymérique,
- A désigne un radical divalent dérivé d'un diisocyanate aliphatique, cycloaliphatique ou aromatique, de préférence un radical divalent dérivé d'un polyméthylène diisocyanate, toluène diisocyanate ou méthanediphénylène diisocyanate;
- $R_1$ et $R_2$, identiques ou différents, désignent un radical alkyle ou alcényle en $C_8$-$C_{30}$, de préférence $C_{10}$-$C_{20}$, et plus particulièrement $C_{12}$-$C_{18}$;
- ai et bi, identiques ou différents, étant tels que la somme ai + bi varie de 20 à 200 moles et de préférence de 60 à 120 moles;
- le rapport molaire ai/bi est compris entre 30/70 et 90/10, de préférence entre 50/50 et 90/10 et plus particulièrement entre 70/30 et 85/15.

Les composés de formule (III) particulièrement préférés sont ceux où A désigne le reste hexaméthylène diisocyanate; R1 et R2 désignent un radical lauryle ou un mélange de radicaux dérivés du suif; les radicaux $Ri[(C_3H_6O)-(C_2H_4O)]_{(bi,ai)}$ ont de préférence un poids moléculaire de l'ordre de 4000 avec Ri désignant R1 ou R2 et ai et bi désignant a et b ou a' et b' définis ci-dessus.

Des composés de formule (III) utilisables selon l'invention sont décrits dans la demande européenne EP 260430 et commercialisés sous la dénomination DAPRAL® T210 et DAPRAL® T212 par la société AKZO.

On préfère particulièrement les polymères du groupe I présentant la formule (IV) suivante :

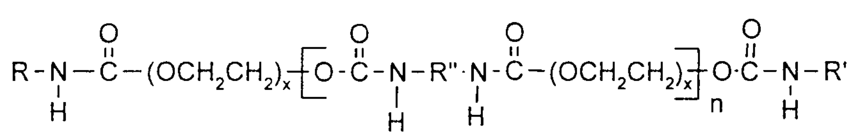

(IV)

dans laquelle:

- R et R', identiques ou différents, sont des radicaux hydrocarbonés en $C_8$-$C_{18}$
- R" est un radical hydrocarboné en $C_7$-$C_{36}$

x varie de 90 à 600

n varie de 1 à 4

Des polymères de ce type sont par exemple le produit BERMODOL® PUR 2130 vendu par la société AKZO NOBEL, les produits proposés sous les dénominations DW1206J®, DW1206B®, DW1206F® par la société SEPPIC, les produits vendus sous les dénominations ACRYSOL® RM-2020, ACRYSOL® RM-8, ACRYSOL® RM-825, ACRYSOL® 44 par la société SEPPIC.

Des compositions cosmétiques en particulier détergentes comprenant des polyuréthanes associatifs tels que définis ci-dessus et des alkylpolyglycosides sont décrites dans la demande de brevet EP 555 155.

Selon l'invention, on peut utiliser tout polymère anionique moussant ou non moussant connu en soi. Bien entendu, on peut utiliser un ou plusieurs polymères anioniques.

Ainsi, les polymères anioniques généralement utilisés sont des polymères comportant des groupements dérivés d'acide carboxylique, sulfonique ou phosphorique et ont un poids moléculaire compris entre environ 500 et 5.000.000.

Les groupements carboxyliques sont apportés par des monomères mono ou diacides carboxyliques insaturés tels que ceux répondant à la formule :

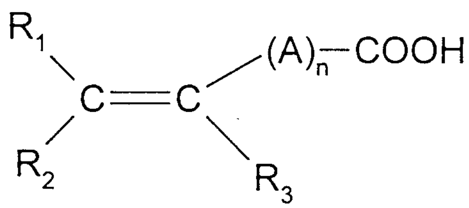

(V)

dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, $R_1$ désigne un atome d'hydrogène, un groupement phényle ou benzyle, $R_2$ désigne un atome d'hydrogène, un groupement alkyle inférieur ou carboxyle, $R_3$ désigne un atome d'hydrogène, un groupement alkyle inférieur, un groupement $-CH_2$ - COOH, phényle ou benzyle.

Dans la formule précitée un radical alkyle inférieur désigne de préférence un groupement ayant 1 à 4 atomes de carbone et en particulier, méthyle et éthyle.

Les polymères anioniques à groupements carboxyliques préférés selon l'invention sont :

A) les homo- ou copolymères d'acide acrylique ou méthacrylique ou leurs sels et en particulier les produits vendus sous les dénominations VERSICOL® E ou K par la société ALLIED COLLOID, ULTRAHOLD® par la société BASF. Les copolymères d'acide acrylique et d'acrylamide vendus sous la forme de leur sel de sodium sous les dénominations RETEN® 421, 423 ou 425 par la Société HERCULES, les sels de sodium des acides polyhydroxycarboxyliques.

B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER® par la Société AMERICAN CYANAMID. On peut également citer les copolymères d'acide acrylique et de méthacrylate d'alkyle en $C_1$-$C_4$ et les terpolymères de vinylpyrrolidone, d'acide acrylique et de méthacrylate d'alkyle en $C_1$-$C_{20}$ par exemple de lauryle tel que celui vendu par la société ISP sous la dénomination ACRYLIDONE® LM. On peut également citer le copolymère d'acide méthacrylique et d'acrylate d'éthyle vendu sous la dénomination LUVIMER® MAEX par la société BASF.

C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique $\alpha$- ou $\beta$-cyclique. De tels polymères sont décrits entre autres dans les brevets français 1.222.944, 1.580.545, 2.265.782, 2.265.781, 1.564.110 et

2.439.798. Des produits commerciaux entrant dans cette classe sont les résines 28-29-30, 26-13-14 et 28-13-10 vendues par la société NATIONAL STARCH.

D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2.047.398, 2.723.248, 2.102.113, le brevet GB 839.805 et notamment ceux vendus sous les dénominations GANTREZ® AN ou ES par la société ISP.
Des polymères entrant également dans cette classe sont les copolymères d'anhydrides maléique, citraconique, itaconique et d'un ester allylique ou méthallylique comportant éventuellement un groupement acrylamide, méthacrylamide, une $\alpha$-oléfine, des esters acryliques ou méthacryliques, des acides acrylique ou méthacrylique ou la vinylpyrrolidone dans leur chaîne, les fonctions anhydrides sont monoestérifiées ou monoamidifiées. Ces polymères sont par exemple décrits dans les brevets français 2.350.384 et 2.357.241 de la demanderesse.

E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.
Ces polymères peuvent être notamment choisis parmi :

- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.

- les sels de l'acide polystyrène sulfonique les sels de sodium ayant un poids moléculaire d'environ 500.000 et d'environ 100.000 vendus respectivement sous les dénominations Flexan® 500 et Flexan® 130 par National Starch. Ces composés sont décrits dans le brevet FR 2.198.719.

- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA® POLYMER HSP 1180 par HENKEL.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD® STRONG par la société BASF, les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ® ES 425 par la société ISP, les copolymères d'acide méthacrylique et de méthacrylate de méthyle vendus sous la dénomination EUDRAGIT® L par la société ROHM PHARMA, le copolymère d'acide méthacrylique et d'acrylate d'éthyle vendu sous la dénomination LUVIMER® MAEX par la société BASF, le terpolymère de vinylpyrrolidone/acide acrylique/méthacrylate de lauryle vendu sous la dénomination ACRYLIDONE® LM par la société ISP et le copolymère acétate de vinyle/acide crotonique vendu sous la dénomination LUVISET® CA 66 par la société BASF et le terpolymère acétate de vinyle/acide crotonique/polyéthylèneglycol sous la dénomination ARISTOFLEX® A par la société BASF.
Les polymères anioniques les plus particulièrement préférés sont choisis parmi le copolymère méthylvinyléther/anhydride maléique mono estérifié vendu sous la dénomination GANTREZ® ES 425 par la société ISP, les copolymères d'acide méthacrylique et de méthacrylate de méthyle vendus sous la dénomination EUDRAGIT® L par la société ROHM PHARMA, le copolymère d'acide méthacrylique et d'acrylate d'éthyle vendu sous la dénomination LUVIMER® MAEX par la société BASF, le terpolymère de vinylpyrrolidone/acide acrylique/méthacrylate de lauryle vendu sous la dénomination ACRYLIDONE® LM par la société ISP.
Selon l'invention, on peut également utiliser des polymères anioniques sous forme de latex ou de pseudolatex, c'est à dire sous forme d'une dispersion de particules de polymères insolubles.
Selon l'invention, le polyuréthanne associatif est généralement présent dans des proportions allant de 0,01% à 5 % en poids environ par rapport au poids total de la composition, et de préférence de 0,05 à 3 % en poids.
Les polymères anioniques sont généralement présents dans des proportions comprises entre 0,1% et 20 % en poids environ par rapport au poids total de la composition, et de préférence entre 0,5% et 8 % en poids environ par rapport au poids total de la composition.

Le milieu cosmétiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Le pH des compositions selon l'invention est généralement compris entre 2 et 9, et en particulier entre 3 et 8. Il peut être ajusté à la valeur choisie au moyen d'agents alcalinisants ou acidifiants habituellement utilisés en cosmétique pour ce type d'application.

Les compositions selon l'invention peuvent encore contenir, des agents épaississants, des agents tensioactifs, des agents conservateurs, des séquestrants, des adoucissants, des colorants, des agents modificateurs de viscosité, des agents nacrants, des agents hydratants, des agents antipelliculaires, des agents antiséborrhéiques, des filtres solaires, des protéines, des vitamines, des plastifiants, des hydroxyacides, des électrolytes et des parfums.

Les compositions selon l'invention peuvent également contenir des agents conditionneurs. Ceux-ci peuvent alors être choisis parmi les huiles et les cires naturelles ou synthétiques, les alcools gras, les esters d'alcools polyhydriques, les glycérides, les huiles, les gommes et résines de silicone, les polymères (cationiques, non ioniques ou amphotères) ou les mélanges de ces différents composés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée : en particulier, ils ne doivent pas empêcher la production de la mousse ni diminuer ses propriétés.

Les agents propulseurs sont généralement présents dans des proportions inférieures à 25% en poids par rapport au poids total de la composition et de préférence dans des proportions comprises entre 1 % et 10 %

L'agent propulseur peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Les compositions introduites dans le dispositif aérosol peuvent par exemple se présenter sous forme de lotion, de dispersions ou d'émulsions qui, après distribution à partir du dispositif aérosol, forment des mousses à appliquer sur les matières kératiniques telles que les cheveux, les cils ou la peau.

L'invention a encore pour objet un procédé de traitement cosmétique des matières kératiniques, telles que les cheveux, caractérisé en ce qu'il consiste à appliquer sur matières kératiniques une composition cosmétique telle que définie précédemment, puis à effectuer éventuellement un rinçage à l'eau, après un éventuel temps de pose.

Ainsi, ce procédé selon l'invention permet le maintien de la coiffure ou des cils, le traitement, le soin de la peau, des cheveux, des cils ou de toute autre matière kératinique.

Les compositions cosmétiques selon l'invention peuvent être utilisées par exemple pour la peau, les cheveux, les cils ou les sourcils et de préférence pour les cheveux.

Les compositions peuvent être plus particulièrement des compositions à rincer ou non, à appliquer avant ou après un shampooing, une coloration, une décoloration, une permanente ou un défrisage.

Les compositions selon l'invention sont encore plus particulièrement utilisées comme compositions de fixation et/ou de coiffage.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

## EXEMPLE 1

On a préparé une mousse de coiffage (A) selon l'invention et deux mousses B et C non conformes à l'invention de composition suivante :

| en gMA | A | B | C |
|---|---|---|---|
| GANTREZ® ES 425 | 1 | 1 | |
| BERMODO®L pur 2130 | 0,2 | | 0,2 |
| AMP qs | pH 7,5 | pH 7,5 | pH 7,5 |
| Parfum, colorant | qs | qs | qs |
| Eau qsp | 100 | 100 | 100 |

Les 3 compositions sont conditionnées de la façon suivante :

<u>Conditionnement en aérosol :</u>

90g de la composition ci-dessus sont conditionnés dans un récipient aérosol en présence de 10g d'un mélange ternaire de n-butane, isobutane et propane, (23/55/22), vendu sous la dénomination de "AEROGAZ® 3,2 N" par la société ELF AQUITAINE

GANTREZ® ES 425 (ISP) : Copolymère méthylvinyléther/monomaléate de butanol

BERMODOL® pur 2130 de AKZO NOBEL : Polyuréthanne associatif

AMP : 2-amino 2-méthyl 1-propanol

Les propriétés de ces 3 mousses ont été évaluées par un panel de 5 testeurs expérimentés, les résultats sont rassemblés dans le tableau ci-dessous.

| Propriétés | A (Invention) | B (Comparatif) | C (Comparatif) |
|---|---|---|---|
| Rigidité | 3,4 | 1,2 | 0,3 |
| Expansion | 5 | 3,2 | 1,8 |
| Stabilité | 5 | 1,2 | 1 |

Notation des propriétés de 0 à 6 :
0 nul
6 excellent

Les propriétés de rigidité, de stabilité et d'expansion de la mousse contenant seulement le polymère anionique sont nettement améliorées par l'addition d'un polyuréthanne associatif.

On a également comparé la mousse (A) à quatre mousses (D, E, F et G) non conformes à l'invention contenant un polyuréthanne non associatif

| en gMA | A | D | E | F | G |
|---|---|---|---|---|---|
| GANTREZ® ES 425 | 1 | 1 | 1 | | |
| BERMODOL® pur 2130 | 0,2 | | | | |
| URAFLEX® XP 402 UZ | | 0,2 | | 0,2 | |
| MELIO PROMUL® 72 | | | 0,2 | | 0,2 |
| AMP qs | pH7,5 | pH7,5 | pH7,5 | pH7,5 | pH7,5 |
| Parfum, colorant | qs | qs | qs | qs | qs |
| Eau qsp | 100 | 100 | 100 | 100 | 100 |

Le conditionnement aérosol est identique pour les 5 mousses et similaire à celui de l'exemple 1.

URAFLEX® XP 402 UZ de DSM Resins : polyuréthanne non associatif
MELIO PROMUL® 72 de QUINN : polyuréthanne non associatif

Les propriétés de ces 5 mousses ont été évaluées par un panel de 5 testeurs expérimentés, les résultats sont rassemblés dans le tableau ci-dessous.

| Propriétés | A (Invention) | D (Comparatif) | E (Comparatif) | F (Comparatif) | G (Comparatif) |
|---|---|---|---|---|---|
| Rigidité | 3,4 | 1,2 | 0 | 0 | 0,2 |
| Expansion | 5 | 3,2 | 2,5 | 1,3 | 1,6 |
| Stabilité | 5 | 1,7 | 0,5 | 0 | 0,8 |

Les propriétés de rigidité, de stabilité et d'expansion de la mousse contenant seulement le polymère anionique ne sont pas améliorées par l'addition d'un polyuréthanne non associatif même lorsque le polyuréthanne non associatif a des propriétés pratiquement identiques à celles du polymère associatif (mousses G et C).

**EXEMPLE 2**

On a préparé une mousse de coiffage de composition suivante :

| | |
|---|---|
| - Copolymère méthylvinyléther/monomaléate de butanol GANTREZ® ES 425 (ISP) | 4 gMA |
| - Polyuréthanne associatif (ACRYSOL® 44 de SEPPIC) | 0,5 gMA |
| - Alcool éthylique | 17,3 g |
| - 2-amino 2-méthyl 1-propanol qs | pH7,5 |
| - Parfum, colorant qs | |
| - Eau déminéralisée qsp | 100g |

Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ® 3,2 N" par la société ELF AQUITAINE

LA mousse présente de bonnes propriétés de rigidité, de stabilité et d'expansion. On a appliqué cette mousse sur des cheveux lavés et essorés. Les cheveux sont ensuite séchés en faisant un brushing. Les cheveux traités avec cette composition selon l'invention sont doux et le toucher de ces derniers est naturel et agréable.

**EXEMPLE 3**

On a préparé une mousse de coiffage de composition suivante :

| | |
|---|---|
| - Copolymère méthylvinyléther/monomaléate de butanol GANTREZ® ES 425 (ISP) | 2 gMA |
| - Polyuréthanne associatif (BERMODOL pur 2130 de AKZO NOBEL) | 0,5 gMA |
| - 2-amino 2-méthyl 1-propanol qs | pH 7,5 |
| - Parfum, colorant qs | |
| - Eau déminéralisée qsp | 100g |

Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ® 3,2 N" par la société ELF AQUITAINE

La composition présente les mêmes propriétés qu'à l'exemple 4.

**EXEMPLE 4**

On a préparé une mousse de coiffage de composition suivante :

| | |
|---|---|
| - Terpolymère acétate de vinyle/acide crotonique/néodécanoate de vinyle (RESINE 28-2930 de NATIONAL STARCH) | 5 gMA |
| - Polyuréthanne associatif (ACRYSOL® 44 de SEPPIC) | 1 gMA |
| - 2-amino 2-méthyl 1-propanol qs | pH7,5 |
| - Parfum, colorant qs | |
| - Eau déminéralisée qsp | 100g |

Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ® 3,2 N" par la société ELF AQUITAINE

La composition présente les mêmes propriétés qu'à l'exemple 4.

**EXEMPLE 5**

On a préparé une mousse de coiffage de composition suivante :

| | |
|---|---|
| - Copolymère méthylvinyléther/monomaléate de butanol GANTREZ® ES 425 (ISP) | 0,5 gMA |
| - Copolymère hydroxyéthylcellulose/chlorure de diallyl diméthyl ammonium vendu sous la dénomination CELQUAT® L 200 par la société NATIONAL STARCH | 0,5 gMA |
| - Polyuréthanne associatif (ACRYSOL® 44 de SEPPIC) | 0,3 gMA |
| - Alcool éthylique | 10 g |
| - 2-amino 2-méthyl 1-propanol | 0,15 g |
| - Parfum, colorant qs | |
| - Eau déminéralisée qsp | 100g |

Conditionnement en aérosol :

90g de la composition ci-dessus sont conditionnés dans un récipient aérosol en présence de 10g de HFC 134A (1,1,1,2-tétrafluoroéthane).

La composition présente les mêmes propriétés qu'à l'exemple 4.

## Revendications

1. Composition cosmétique pressurisée en aérosol en présence d'un agent propulseur et susceptible de former une mousse, caractérisée par le fait qu'elle comprend dans un milieu aqueux cosmétiquement acceptable, au moins un polyuréthanne associatif lequel contient au moins une séquence hydrophile, au moins une séquence hydrophobe et au moins un groupement uréthanne, et au moins un polymère anionique, l'un au moins des deux polymères étant un polymère à pouvoir moussant.

2. Composition selon la revendication précédente, caractérisée par le fait que le polymère à pouvoir moussant est le polyuréthanne associatif.

3. Composition selon l'une quelconque des revendications précédentes caractérisée par le fait que le polyuréthanne associatif et le polymère anioniques sont des polymères à pouvoir moussant.

4. Composition selon l'une quelconque des revendications précédentes, caractérisée par le fait que la séquence hydrophile est une séquence polyoxyalkylénée et en particulier polyoxyéthylénée.

5. Composition selon l'une quelconque des revendications précédentes, caractérisée par le fait que la séquence hydrophobe est une chaîne grasse comprenant de 8 à 30 atomes de carbone.

6. Composition selon l'une quelconque des revendications précédentes, caractérisée par le fait que le polyuréthanne associatif comporte au moins deux séquence hydrophobes.

7. Composition selon l'une quelconque des revendications précédentes caractérisée par le fait que le polyuréthanne associatif est choisi parmi les trois groupes suivants :

   <u>Groupe I</u>
   Les polyuréthannes répondant à la formule (I) suivante :

   $$X\text{-}B_p\text{-}E_q\text{-}(B\text{-}E)_n\text{-}B_r\text{-}E_t\text{-}X \qquad (I)$$

   dans laquelle,

   n est un nombre compris entre 1 et 10, p, q, r et t, identiques ou différents, valent 0 ou 1, avec au moins q ou r valant 1 et, t valant 0 lorsque r est égal à 0,

   sous réserve que:

   lorsque q vaut 1, alors :

   a) p = r = t = 0 , ou
   b) p = 0 et r = t = 1 , ou
   c) t = 0 et r = p = 1 , et

   lorsque q vaut 0, alors :

   r = 1 et p = t = 0 ;

   <u>Groupe II</u>
   Les polyuréthannes répondant à la formule (II) suivante :

   $$(H\text{-}E\text{-}OCH_2)_sL[Q_v\text{-}(D_u\text{-}E\text{-}X)_w\text{-}R_z]_m \qquad (II)$$

   dans laquelle,

   m est un nombre entier variant de 2 à 4 et s un nombre entier variant de 0 à 2, la somme de m et s variant de 2 à 4, w est un nombre entier variant de 1 à 3, et chacun de u, v et z est, indépendamment l'un de l'autre, 0 ou 1 ; L représente Y, Z ou -O-, Y étant un radical hydrocarboné hydrophobe contenant au moins un atome de carbone et préférence 1 à 4 atomes de carbone, et Z un radical hydrophobe trivalent choisi

parmi les groupements suivants :
$-OCONH(CH_2)_6N[CONH(CH_2)_6NHCOO-]_2$,
$CH_3C[CH_2O-OCNHC_7H_6NHCO-]_3$, et
$CH_3CH_2C[CH_2O-OCNHC_7H_6NHCO-]_3$
Q représente le groupe -CH2C-, et D le groupe -CH2O-, sous réserve que,

a) lorsque L représente Y, alors u et w valent chacun 1, v et z valent 0, m vaut au moins 2 et la somme de m et s est 4 ;
b) lorsque L vaut Z, alors u, v et s valent chacun 0, m vaut 3, w vaut 2 ou 3 et z est 0 ou 1 ; et
c) lorsque L représente -O-, alors v et u valent chacun 1, w varie de 1 à 3, m vaut 2, et s et z valent chacun 0 ;

dans chacune des formules de ces deux groupes, X et R représentent un radical hydrophobe; B un groupe divalent hydrophobe de formule -CONH-G-NHCO-O-, dans laquelle G est un radical divalent dérivé d'un di- ou d'un triisocyanate organique dont tous les groupes isocyanate ont réagi; et E représente un groupe poyéther divalent non ionique.

Groupe III
Les polyuréthannes de ce groupe sont obtenus par réaction (a) d'un réactif polyfonctionnel choisi parmi les polyols organiques possédant au moins trois groupements hydroxyle, les polyisocyanates organiques possédant au moins trois groupements isocyanates, et leur mélange ; (b) un réactif difonctionnel choisi parmi les diols organiques, les diisocyanates organiques, et leur mélange, le diol étant présent dans le mélange de la réaction lorsque le polyisocyanate est présent et le diisocyanate étant présent lorsque le polyol est présent ; (c) un composé monofonctionnel hydroxyl ou amino en une quantité suffisante pour piéger tout groupement isocyanate n'ayant pas réagi lors de la réaction entre (a) et (b), et pour prévenir la coagulation du mélange réactionnel ; et éventuellement (d) un monoisocyanate organique pour piéger les groupements hydroxyl restant après la réaction entre (a) et (b) ; réaction dans laquelle, au moins l'un du polyol et du diol contient au moins un segment poyéther soluble dans l'eau d'un poids moléculaire d'au moins 1500, la somme des atomes de carbone dans les réactifs contenant des groupements isocyanate, des groupements hydroxyl et des groupements amino, est d'au moins 20, et le poids moléculaire moyen des composants de la composition est d'environ 10 000 à 200 000.

8. Composition selon la revendication précédente, caractérisée par le fait que les polyuréthannes associatifs présentent la formule suivante (IV) :

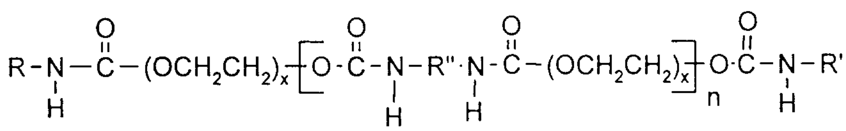

(IV)

dans laquelle:

R et R', identiques ou différents, sont des radicaux hydrocarbonés en $C_8$-$C_{18}$
R'' est un radical hydrocarboné en $C_7$-$C_{36}$
x varie de 90 à 600
n varie de 1 à 4

9. Composition selon l'une quelconque des revendications 1 à 5, caractérisée en ce que le polyuréthanne associatif répond à la formule (III) suivante:

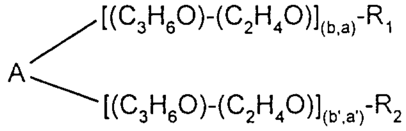

(III)

dans laquelle,

$[(C_3H_6O)-(C_2H_4O)]_{(bi,ai)}$ , avec (bi,ai) désignant (b,a) ou (b',a'), signifie qu'il s'agit d'un polymère statistique d'oxyde de propylène et d'oxyde d'éthylène contenant a mole d'oxyde d'éthylène et b mole d'oxyde de propylène réparties de manière aléatoire dans la chaîne polymérique,
A désigne un radical divalent dérivé d'un diisocyanate aliphatique, cycloaliphatique ou aromatique;
$R_1$ et $R_2$, identiques ou différents, désignent un radical alkyle ou alcényle en $C_8$-$C_{30}$, de préférence $C_{10}$-$C_{20}$;
ai et bi, identiques ou différents, étant tels que la somme ai + bi varie de 20 à 200 moles et de préférence de 60 à 120 moles;
le rapport molaire ai/bi est compris entre 30/70 et 90/10.

**10.** Composition selon l'une des revendications précédentes, caractérisée par le fait que le polymère anionique est choisi parmi :

- les polymères comportant des motifs carboxyliques dérivant de monomères mono ou diacides carboxyliques insaturés de formule :

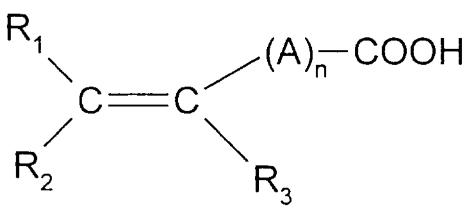

(V)

dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, $R_1$ désigne un atome d'hydrogène, un groupement phényle ou benzyle, $R_2$ désigne un atome d'hydrogène, un groupement alkyle inférieur ou carboxyle, $R_3$ désigne un atome d'hydrogène, un groupement alkyle inférieur, un groupement $-CH_2$ - COOH, phényle ou benzyle.
- les polymères comprenant des motifs dérivant d'acide sulfonique tels que des motifs vinylsulfonique, styrènesulfonique, acrylamido alkylsulfonique.

**11.** Composition selon la revendication 10, caractérisée en ce que le polymère anionique est choisi parmi :

A) les homo- ou copolymères d'acide acrylique ou méthacrylique ou leurs sels, les copolymères d'acide acrylique et d'acrylamide et leurs sels, les sels de sodium d'acides polyhydroxycarboxyliques.

B) Les copolymères des acides acrylique ou méthacryliques avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en $C_1$-$C_4$ et les terpolymères de vinylpyrrolidone, d'acide acrylique et de méthacrylate d'alkyle en $C_1$-$C_{20}$.

C) les copolymères dérivés d'acide crotonique tels que ceux comportant dans leur chaîne des motif acétate ou propionate de vinyle et éventuellement d'autres monomères tels que esters allylique ou méthallylique, éther

vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés.

D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées.

E) les polyacrylamides comportant des groupements carboxylates.

**12.** Composition selon la revendication 11, caractérisée en ce que le polymère anionique est choisi parmi :

- les copolymères d'acide acrylique tels que le terpolymère acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié.
- les copolymères d'acide méthacrylique et de méthacrylate de méthyle;
- le copolymère d'acide méthacrylique et d'acrylate d'éthyle;
- le terpolymère de vinylpyrrolidone/acide acrylique/méthacrylate de lauryle ;
- le copolymère acétate de vinyle/acide crotonique
- le terpolymère acétate de vinyle/acide crotonique/polyéthylèneglycol.

**13.** Composition selon l'une quelconque des revendications précédentes, caractérisée par le fait que le polyuréthanne associatif est présent dans des proportions allant de 0,01% à 5 % en poids environ par rapport au poids total de la composition.

**14.** Composition selon la revendication 13, caractérisée par le fait que polyuréthanne associatif est présent dans des proportions allant de 0,05 à 3 % en poids par rapport au poids total de la composition.

**15.** Composition selon l'une quelconque des revendications précédentes, caractérisée par le fait que les polymères anioniques sont présents dans des proportions comprises entre 0,1% et 20 % en poids environ par rapport au poids total de la composition.

**16.** Composition selon la revendication 15, caractérisée par le fait que les polymères anioniques sont présents dans des proportions comprises entre 0,5% et 8 % en poids environ par rapport au poids total de la composition.

**17.** Composition selon l'une quelconque des revendications précédentes caractérisée par le fait que le milieu aqueux cosmétiquement acceptable contient de l'eau ou un mélange d'eau et d'un solvant cosmétiquement acceptable.

**18.** Composition cosmétique sous forme de mousse, caractérisée par le fait qu'elle résulte de l'expansion à l'air d'une composition telle que définie dans l'une quelconque des revendications précédentes.

**19.** Procédé de traitement cosmétique des matières kératiniques, telles que les cheveux, caractérisé en ce qu'il consiste à appliquer sur les matières kératiniques une composition cosmétique telle que définie précédemment selon l'une des revendications 1 à 18, puis à effectuer éventuellement un rinçage à l'eau, après un éventuel temps de pose.

**20.** Utilisation d'un polyuréthanne associatif lequel contient au moins une séquence hydrophile, au moins une séquence hydrophobe et au moins un groupement uréthanne, pour améliorer les propriétés des mousses résultant de l'expansion à l'air d'une composition pressurisée en aérosol à base de polymère anionique et d'agent propulseur, l'un au moins des deux polymères étant un polymère à pouvoir moussant.

## Claims

1. Cosmetic composition pressurized as an aerosol in the presence of a propellant and capable of forming a mousse, characterized in that it comprises, in a cosmetically acceptable aqueous medium, at least one associative polyurethane which contains at least one hydrophilic sequence, at least one hydrophobic sequence and at least one urethane group, and at least one anionic polymer, at least one of the two polymers being a polymer with foaming power.

2. Composition according to the preceding claim, characterized in that the polymer with foaming power is the associative polyurethane.

3. Composition according to either of the preceding claims, characterized in that the associative polyurethane and the anionic polymer are polymers with foaming power.

4. Composition according to any one of the preceding claims, characterized in that the hydrophilic sequence is a polyoxyalkylenated and in particular polyoxyethylenated sequence.

5. Composition according to any one of the preceding claims, characterized in that the hydrophobic sequence is a fatty chain comprising from 8 to 30 carbon atoms.

6. Composition according to any one of the preceding claims, characterized in that the associative polyurethane contains at least two hydrophobic sequences.

7. Composition according to any one of the preceding claims, characterized in that the associative polyurethane is chosen from the following three groups:

Group I

Polyurethanes corresponding to the following formula (I):

$$X\text{-}B_p\text{-}E_q\text{-}(B\text{-}E)_n\text{-}B_r\text{-}E_t\text{-}X \qquad (I)$$

in which

n is a number between 1 and 10, p, q, r and t, which may be identical or different, are equal to 0 or 1, with at least q or r equal to 1 and, t is equal to 0 when r is equal to 0,
with the proviso that:

when q is equal to 1, then:

a) $p = r = t = 0$ , or
b) $p = 0$ and $r = t = 1$ , or
c) $t = 0$ and $r = p = 1$ , and

when q is equal to 0, then:

$r = 1$ and $p = t = 0$ ;

Group II

Polyurethanes corresponding to the following formula (II):

$$(H\text{-}E\text{-}OCH_2)_sL[Q_v\text{-}(D_u\text{-}E\text{-}X)_w\text{-}R_z]_m \qquad (II)$$

in which

m is an integer ranging from 2 to 4 and s is an integer ranging from 0 to 2, the sum of m and s ranging from 2 to 4, w is an integer ranging from 1 to 3 and each of u, v and z is, independently of each other, 0 or 1; L represents Y, Z or -O-, Y being a hydrophobic hydrocarbon radical containing at least one carbon atom and preferably from 1 to 4 carbon atoms, and Z is a trivalent hydrophobic radical chosen from the following groups:

$-OCONH(CH_2)_6N[CONH(CH_2)_6NHCOO-]_2$,
$CH_3C[CH_2O-OCNHC_7H_6NHCO-]_3$, and
$CH_3CH_2C[CH_2O-OCNHC_7H_6NHCO-]_3$
Q represents the $-CH_2C-$ group and D the $-CH_2O-$ group, with the proviso that

a) when L represents Y, then u and w are each equal to 1, v and z are equal to 0, m is equal to at least 2 and the sum of m and s is 4;
b) when L is equal to Z, then u, v and s are each equal to 0, m is equal to 3, w is equal to 2 or 3 and z is 0 or 1; and
c) when L represents -O-, then v and u are each equal to 1, w ranges from 1 to 3, m is equal to 2 and s and z are each equal to 0;
in each of the formulae of these two groups, X and R represent a hydrophobic radical; B represents a hydrophobic divalent group of formula -CONH-G-NHCO-O-, in which G is a divalent radical derived from an organic di- or triisocyanate in which all the isocyanate groups have reacted; and E represents a nonionic divalent polyether group;

<u>Group III</u>
The polyurethanes of this group are obtained by reaction (a) of a polyfunctional reactant chosen from organic polyols possessing at least three hydroxyl groups, organic polyisocyanates possessing at least three isocyanate groups, and a mixture thereof; (b) a difunctional reactant chosen from organic diols, organic diisocyanates and a mixture thereof, the diol being present in the reaction mixture when the polyisocyanate is present and the diisocyanate being present when the polyol is present; (c) a hydroxyl or amino monofunctional compound in a sufficient amount to trap any isocyanate group which has not reacted during the reaction between (a) and (b), and in order to prevent coagulation of the reaction mixture; and, optionally, (d) an organic monoisocyanate in order to trap the hydroxyl groups remaining after the reaction between (a) and (b); in which reaction, at least either the polyol or the diol contains at least one water-soluble polyether segment with a molecular weight of at least 1500, the sum of the carbon atoms in the reactants containing isocyanate groups, hydroxyl groups and amino groups is at least 20, and the average molecular weight of the components of the composition is approximately from 10,000 to 200,000.

8. Composition according to the preceding claim, characterized in that the associative polyurethanes have the following formula (IV):

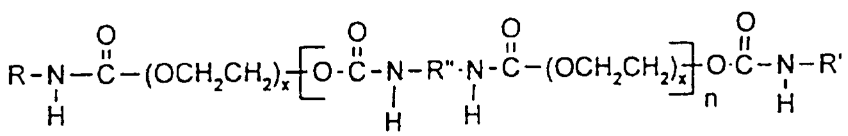
(IV)

in which:

R and R', which may be identical or different, are $C_8$-$C_{18}$ hydrocarbon radicals
R" is a $C_7$-$C_{36}$ hydrocarbon radical
x ranges from 90 to 600
n ranges from 1 to 4.

9. Composition according to any one of Claims 1 to 5, characterized in that the associative polyurethane corresponds to the following formula (III):

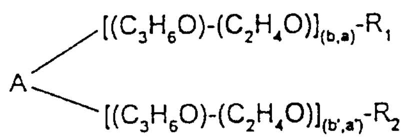
(III)

in which

$[(C_3H_6O)-(C_2H_4O)]_{(bi,ai)}$, with (bi,ai) denoting (b,a) or (b',a'), means that it is a statistical polymer of propylene oxide and ethylene oxide containing a mol of ethylene oxide and b mol of propylene oxide distributed randomly in the polymer chain,

A denotes a divalent radical derived from an aliphatic, cycloaliphatic or aromatic diisocyanate,

$R_1$ and $R_2$, which may be identical or different, denote a $C_8$-$C_{30}$, preferably $C_{10}$-$C_{20}$, alkyl or alkenyl radical;

ai and bi, which may be identical or different, being such that the sum ai + bi ranges from 20 to 200 mol and preferably from 60 to 120 mol;

the ai/bi molar ratio is between 30/70 and 90/10.

**10.** Composition according to one of the preceding claims, characterized in that the anionic polymer is chosen from:

- polymers containing carboxylic units derived from unsaturated monocarboxylic or dicarboxylic acid monomers of formula:

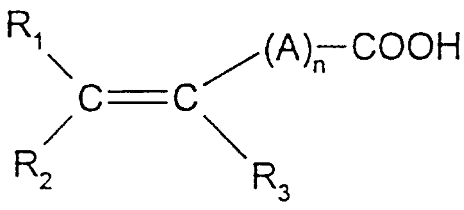

(V)

in which n is an integer from 0 to 10, A denotes a methylene group, optionally connected to the carbon atom of the unsaturated group or to the neighbouring methylene group when n is greater than 1 via a hetero atom such as oxygen or sulphur, $R_1$ denotes a hydrogen atom or a phenyl or benzyl group, $R_2$ denotes a hydrogen atom or a lower alkyl or carboxyl group, $R_3$ denotes a hydrogen atom, a lower alkyl group or a $-CH_2$-COOH, phenyl or benzyl group.
- polymers comprising units derived from sulphonic acid, such as vinylsulphonic, styrenesulphonic or acrylamidoalkylsulphonic units.

**11.** Composition according to Claim 10, characterized in that the anionic polymer is chosen from:

A) Homo- or copolymers of acrylic or methacrylic acid or salts thereof, the copolymers of acrylic acid and acrylamide and salts thereof, and the sodium salts of polyhydroxycarboxylic acids.

B) Copolymers of acrylic or methacrylic acid with a monoethylenic monomer such as ethylene, styrene, vinyl esters or acrylic or methacrylic acid esters, optionally grafted onto a polyalkylene glycol such as polyethylene glycol and optionally crosslinked; copolymers of this type containing an optionally N-alkylated and/or hydroxyalkylated acrylamide unit in their chain, the copolymers of acrylic acid and $C_1$-$C_4$ alkyl methacrylate and the terpolymers of vinylpyrrolidone, acrylic acid and methacrylate of $C_1$-$C_{20}$ alkyl.

C) Copolymers derived from crotonic acid, such as those containing in their chain vinyl acetate or propionate units and optionally other monomers such as allylic or methallylic esters, vinyl ether or vinyl ester of a linear or branched saturated carboxylic acid containing a long hydrocarbon chain, such as those containing at least 5 carbon atoms, it being possible for these polymers to be optionally grafted and crosslinked.

D) Polymers derived from maleic, fumaric or itaconic acid or anhydride with vinyl esters, vinyl ethers, vinyl halides, phenylvinyl derivatives, acrylic acid and its esters; the copolymers of maleic, citraconic or itaconic anhydride and an allylic or methallylic ester optionally containing an acrylamide or methacrylamide group, an $\alpha$-olefin, acrylic or methacrylic esters, acrylic or methacrylic acid or vinylpyrrolidone in their chain; the anhydride functions are monoesterified or monoamidated.

E) Polyacrylamides containing carboxylate groups.

**12.** Composition according to Claim 11, characterized in that the anionic polymer is chosen from:

- acrylic acid copolymers such as the acrylic acid/ethyl acrylate/N-tert-butyl acrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acid or anhydride with vinyl esters, vinyl ethers, vinyl halides, phenylvinyl derivatives or acrylic acid and its esters, such as methyl vinyl ether/monoesterified maleic anhydride copolymers;

- copolymers of methacrylic acid and methyl methacrylate;
- the copolymer of methacrylic acid and ethyl acrylate;
- the terpolymer of vinylpyrrolidone/acrylic acid/lauryl methacrylate;
- the vinyl acetate/crotonic acid copolymer;
- the vinyl acetate/crotonic acid/polyethylene glycol terpolymer.

**13.** Composition according to any one of the preceding claims, characterized in that the associative polyurethane is present in proportions ranging from 0.01 % to 5 % by weight approximately relative to the total weight of the composition.

**14.** Composition according to Claim 13, characterized in that the associative polyurethane is present in proportions ranging from 0.05 to 3 % by weight relative to the total weight of the composition.

**15.** Composition according to any one of the preceding claims, characterized in that the anionic polymers are present in proportions of between 0.1 % and 20 % by weight approximately relative to the total weight of the composition.

**16.** Composition according to Claim 15, characterized in that the anionic polymers are present in proportions of between 0.5 % and 8 % by weight approximately relative to the total weight of the composition.

**17.** Composition according to any one of the preceding claims, characterized in that the cosmetically acceptable aqueous medium contains water or a mixture of water and a cosmetically acceptable solvent.

**18.** Cosmetic composition in the form of a mousse, characterized in that it results from the expansion into the air of a composition as defined in any one of the preceding claims.

**19.** Process for the cosmetic treatment of keratin substances, such as the hair, characterized in that it consists in applying a cosmetic composition as defined above, according to one of Claims 1 to 18, to the keratin substances and then in optionally rinsing with water, optionally after having been left to stand on the keratin substances for a certain period.

**20.** Use of an associative polyurethane which contains at least one hydrophilic sequence, at least one hydrophobic sequence and at least one urethane group, in order to improve the properties of the mousses resulting from the expansion into the air of a composition pressurized as an aerosol based on anionic polymer and propellant, at least one of the two polymers being a polymer with foaming power.

**Patentansprüche**

**1.** Kosmetische Zusammensetzung, die in Gegenwart eines Treibmittels als Aerosol unter Druck steht und die zur Schaumbildung befähigt ist, dadurch gekennzeichnet, daß sie in einem kosmetisch akzeptablen wässerigen Medium enthält:

- mindestens ein assoziatives Polyurethan, das mindestens eine hydrophile Sequenz, mindestens eine hydrophobe Sequenz und mindestens eine Urethangruppe aufweist, und
- mindestens ein anionisches Polymer,

wobei mindestens eines der beiden Polymeren ein Polymer mit Schaumbildungsvermögen ist.

**2.** Zusammensetzung nach dem vorhergehenden Anspruch, dadurch gekennzeichnet, daß das assoziative Polyurethan das Polymer mit Schaumbildungsvermögen ist.

**3.** Zusammensetzung nach einem der vorhergehenden Ansprüche, dadurch gekennzeichnet, daß das assoziative Polyurethan und das anionische Polymer Polymere mit Schaumbildungsvermögen sind.

**4.** Zusammensetzung nach einem der vorhergehenden Ansprüche, dadurch gekennzeichnet, daß die hydrophile Sequenz eine Polyoxyalkylen-Sequenz und insbesondere eine Polyoxyethylen-Sequenz ist.

**5.** Zusammensetzung nach einem der vorhergehenden Ansprüche, dadurch gekennzeichnet, daß die hydrophobe Sequenz eine Fettsäurekette mit 8 bis 30 Kohlenstoffatomen ist.

6. Zusammensetzung nach einem der vorhergehenden Ansprüche, dadurch gekennzeichnet, daß das assoziative Polyurethan mindestens zwei hydrophobe Sequenzen aufweist.

7. Zusammensetzung nach einem der vorhergehenden Ansprüche, dadurch gekennzeichnet, daß das assoziative Polyurethan ausgewählt ist aus den folgenden drei Gruppen:

Gruppe 1
Polyurethane der folgenden Formel (I):

$$X\text{-}B_p\text{-}E_q\text{-}(B\text{-}E)_n\text{-}B_r\text{-}E_t\text{-}X \quad (I),$$

in der bedeuten:

- n eine Zahl von 1 bis 10,
- p, q, r und t, die gleich oder verschieden sein können, Null oder 1, wobei zumindest q oder r gleich 1 ist und t gleich Null ist, wenn r gleich Null ist,

mit der Maßgabe, daß,

wenn q gleich 1 ist:

a) p = r = t = 0 oder
b) p = 0 und r = t = 1 oder
c) t = 0 und r = p = 1 und,

wenn q gleich Null ist:

r = 1 und p = t = 0 .

Gruppe II
Polyurethane der folgenden Formel (II):

$$(H\text{-}E\text{-}OCH_2)_sL[Q_v\text{-}(D_u\text{-}E\text{-}X)_w\text{-}R_z]_m \quad (II),$$

in der bedeuten:

- m eine ganze Zahl von 2 bis 4 und s Null, 1 oder 2, wobei die Summe von m und s 2 bis 4 beträgt,
- w eine ganze Zahl von 1 bis 3 und
- u, v und z unabhängig voneinander jeweils gleich Null oder 1;
- L Y, Z oder -O-, wobei Y eine hydrophobe Kohlenwasserstoffgruppe ist, die mindestens 1 und vorzugsweise 1 bis 4 Kohlenstoffatome enthält, und Z eine dreiwertige hydrophobe Gruppe darstellt, die ausgewählt ist unter folgenden Gruppen:
  $-OCONH(CH_2)_6N[CONH(CH_2)_6NHCOO-]_2$,
  $CH_3C[CH_2O\text{-}OCNHC_7H_6NHCO-]_3$ und
  $CH_3CH_2C[CH_2O\text{-}OCNHC_7H_6NHCO-]_3$;
- Q die Gruppe $-CH_2C-$ und
- D die Gruppe $-CH_2O-$,
  mit der Maßgabe, daß,

  a) wenn L Y darstellt, u und w jeweils gleich 1 sind, v und z gleich Null sind, m mindestens gleich 2 ist und die Summe von m und s gleich 4 ist;
  b) wenn L Z darstellt, u, v und s jeweils gleich Null sind, m gleich 3 w gleich 2 oder 3 ist und z gleich Null oder 1 ist und,
  c) wenn L -O- darstellt, v und u jeweils gleich 1 sind, w 1 bis 3 beträgt, m gleich 2 ist und s und z jeweils gleich Null sind, wobei in den Formeln dieser beiden Gruppen X und R jeweils eine hydrophobe Gruppe darstellen, B eine zweiwertige hydrophobe Gruppe der Formel -CONH-G-NHCO-O-bedeutet, in der G eine von einem organischen Di- oder Triisocyanat abgeleitete Gruppe ist, bei der alle Isocyanateinheiten umgesetzt wurden, und E eine nichtionische zweiwertige Polyethergruppe darstellt.

Gruppe III

Polyurethane, die erhalten sind durch Umsetzung folgender Reaktanten:

(a) eines polyfunktionellen Reaktanten, der ausgewählt ist unter organischen Polyolen mit mindestens drei Hydroxylgruppen, organischen Polyisocyanaten mit mindestens drei Isocyanatgruppen und Gemischen dieser Verbindungen,

(b) eines difunktionellen Reaktanten, der ausgewählt ist unter organischen Diolen, organischen Diisocyanaten und Gemischen dieser Verbindungen, wobei im Reaktionsgemisch ein Diol eingesetzt wird, wenn als polyfunktioneller Reaktant ein Polyisocyanat eingesetzt wird, und ein Diisocyanat eingesetzt wird, wenn als polyfunktioneller Reaktant ein Polyol eingesetzt wird;

(c) einer monofunktionellen Hydroxyl- oder Aminoverbindung in einer Menge, die ausreicht, um alle bei der Reaktion von (a) mit (b) nicht umgesetzten Isocyanatgruppen abzufangen und die Koagulation des Reaktionsgemischs zu verhindern; sowie gegebenenfalls

(d) eines organischen Monoisocyanats zum Abfangen der nach der Reaktion von (a) mit (b) noch verbliebenen Hydroxylgruppen;

wobei in dieser Reaktion zumindest das Polyol oder das Diol mindestens ein wasserlösliches Polyether-Segment eines Molekulargewichts von mindestens 1500 aufweist, die Summe der Kohlenstoffatome der Reaktanten mit Isocyanat-, Hydroxyl- und Aminogruppen mindestens 20 beträgt und das mittlere Molekulargewicht der Bestandteile der Zusammensetzung etwa 10 000 bis 200 000 beträgt.

8. Zusammensetzung nach dem vorhergehenden Anspruch, dadurch gekennzeichnet, daß die assoziativen Polyurethane der folgenden Formel (IV) entsprechen:

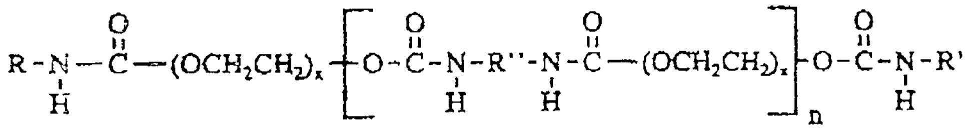

(IV),

in der bedeuten;

- R und R', die gleich oder verschieden sein können, $C_{8-18}$-Kohlenwasserstoffgruppen,
- R'' eine $C_{7-36}$-Kohlenwasserstoffgruppe,
- x 90 bis 600 und
- n 1 bis 4.

9. Zusammensetzung nach einem der Asprüche 1 bis 5, dadurch gekennzeichnet, daß das assoziative Polyurethan der folgenden Formel (III) entspricht:

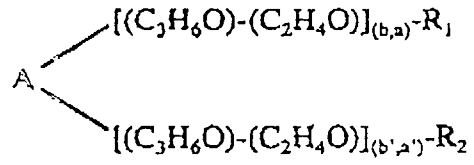

(III),

in der bedeuten:

- $[(C_3H_6O)-(C_2H_4O)]_{(bi,ai)}$ mit (bi,ai) gleich (b,a) oder (b',a'), wobei es sich um ein statistisches Copolymer von Propylenoxid und Ethylenoxid handelt, das a mol Ethylenoxid und b mol Propylenoxid enthält, die statistisch in der Polymerkette verteilt sind,
- A eine von einein aliphatischen, cycloaliphatischen oder aromatischen Diisocyanat abgeleitete zweiwertige Gruppe;
- $R_1$ und $R_2$, die gleich oder verschieden sein können, eine $C_{8-30}$-Alkyl- oder Alkenylgruppe und vorzugsweise eine $C_{10-20}$-Alkyl- oder Alkenylgruppe;
- ai und bi, die gleich oder verschieden sein können, solche Werte, daß die Summe ai + bi 20 bis 200 mol und

vorzugsweise 60 bis 120 mol beträgt und

- das Molverhältnis ai/bi 30/70 bis 90/10 beträgt.

**10.** Zusammensetzung nach einem der vorhergehenden Ansprüche, dadurch gekennzeichnet, daß das anionische Polymer ausgewählt ist unter:

- Polymeren, die Einheiten mit Carboxylfunktionen aufweisen, die sich von monomeren ungesättigten Mono- oder Dicarbonsäuren der folgenden Formel ableiten:

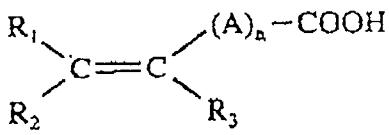

(V),

in der bedeuten:

- n Null oder eine ganze Zahl von 1 bis 10,
- A eine Methylengruppe, die gegebenenfalls entweder an das Kohlenstoffatom der ungesättigten Gruppe oder, wenn n größer als 1 ist; über ein Heteroatom wie Sauerstoff oder Schwefel an die benachbarte Methylengruppe gebunden ist;
- $R_1$ ein Wasserstoffatom, eine Phenyl- oder eine Benzylgruppe,
- $R_2$ ein Wasserstoffatom, eine niedere Alkyl- oder eine Carboxylgruppe und
- $R_3$ ein Wasserstoffatom, eine niedere Alkylgruppe, eine Gruppe $-CH_2-COOH$, eine Phenyl- oder eine Benzylgruppe

sowie

- Polymeren, die von einer Sulfonsäure abgeleitete Einheiten, wie Vinylsulfonsäure-, Styrolsulfonsäure- und Acrylamidoalkylsulfonsäureeinheiten, enthalten.

**11.** Zusammensetzung nach Anspruch 10, dadurch gekennzeichnet, daß das anionische Polymer ausgewählt ist unter:

A) Homo- und Copolymeren von Acrylsäure und Methacrylsäure und ihren Salzen, Copolymeren von Acrylsäure und Acrylamid und ihren Salzen sowie den Natriumsalzen von Polyhydroxycarbonsäuren;

B) Copolymeren von Acrylsäure und Methacrylsäure mit einem einfach ethylenisch ungesättigten Monomer, wie Ethylen, Styrol, Vinylestern, Acrylsäure- und Methacrylsäureestern, die gegebenenfalls auf ein Polyalkylenglykol, wie Polyethylenglykol, gepfropft und gegebenenfalls vernetzt sind; den Copolymeren dieses Typs, die in ihrer Kette eine gegebenenfalls N-alkylierte und/oder N-hydroxyalkylierte Acrylamidfunktion aufweisen, Copolymeren von Acrylsäure mit $C_{1-4}$-Alkylmethacrylaten sowie Terpolymeren von Vinylpyrrolidon mit Acrylsäure und $C_{1-20}$-Alkylmethacrylaten;

C) von Crotonsäure abgeleiteten Copolymeren, wie Copolymeren, die in ihrer Kette Vinylacetat- oder Vinylpropionatfunktionen sowie gegebenenfalls weitere Monomereinheiten aufweisen, wie Allyl- und Methallylester, Vinylether und Vinylester einer geradkettigen oder verzweigten gesättigten Carbonsäure mit langer Kohlenwasserstoffkette, wie z.B. mit mindestens 5 Kohlenstoffatomen, wobei diese Polymeren gegebenenfalls gepfropft und vernetzt sind;

D) von Maleinsäure, Fumarsäure und Itaconsäure sowie den Anhydriden dieser Säuren abgeleiteten Copolymeren mit Vinylestern, Vinylethern, Vinylhalogeniden, Phenylvinylderivaten und Acrylsäure und ihren Estern; Copolymeren von Maleinsäureanhydrid, Citraconsäureanhydrid und Itaconsäureanhydrid mit einem Allyl- oder Methallylester, der gegebenenfalls eine Acrylamidgruppe oder eine Methacrylamidgruppe, ein α-Olefin, Acryl- oder Methacrylsäureester, Acrylsäure oder Methacrylsäure oder Vinylpyrrolidon in seiner Kette aufweist, wobei die Anhydridfunktionen als Monoester oder als Monoamide vorliegen;

E) Polyacrylamiden, die Carboxylatgruppen aufweisen.

**12.** Zusammensetzung nach Anspruch 11, dadurch gekennzeichnet, daß das anionische Polymer ausgewählt ist unter:

- Copolymeren von Acrylsäure, wie Acrylsäure-Ethylacrylat-N-tert-Butyl-acrylamid-Terpolymeren;
- von Crotonsäure abgeleiteten Copolymeren, wie Vinylacetat-Vinyl-tert-butylbenzoat-Crotonsäure-Terpolymeren und Crotonsäure-Vinylacetat-Vinylneododecanoat-Terpolymeren;
- vor Maleinsäure, Fumarsäure und Itaconsäure sowie den Anhydriden dieser Säuren abgeleiteten Copolymeren mit Vinylestern, Vinylethern, Vinylhalogeniden, Phenylvinylderivaten und Acrylsäure und ihren Estern, wie Methylvinylether-Maleinsäureanhydrid-monoester-Copolymeren;
- Copolymeren von Methacrylsäure und Methylmethacrylat;
- Methacrylsäure-Ethylacrylat-Copolymeren;
- Vinylpyrrolldon-Acrylsäure-Laurylmethacrylat-Terpolymeren;
- Vinylacetat-Crotonsäure-Copolymeren und
- Vinylacetat-Crotonsäure-Polyethylenglykol-Terpolymeren.

**13.** Zusammensetzung nach einem der vorhergehenden Ansprüche, dadurch gekennzeichnet, daß das assoziative Polyurethan in einem Mengenanteil von 0,01 bis 5 Gew.-%, bezogen auf das Gesamtgewicht der Zusammensetzung, vorliegt.

**14.** Zusammensetzung nach Anspruch 13, dadurch gekennzeichnet, daß das assoziative Polyurethan in einem Mengenanteil von 0,05 bis 3 Gew.-%, bezogen auf das Gesamtgewicht der Zusammensetzung, vorliegt.

**15.** Zusammensetzung nach einem der vorhergehenden Ansprüche, dadurch gekennzeichnet, daß die anionischen Polymeren in einem Mengenanteil von 0,1 bis 20 Gew.-%, bezogen auf das Gesamtgewicht der Zusammensetzung, vorliegen.

**16.** Zusammensetzung nach Anspruch 15, dadurch gekennzeichnet, daß die anionischen Polymeren in einem Mengenanteil von 0,5 bis 8 Gew.-%, bezogen auf das Gesamtgewicht der Zusammensetzung, vorliegen.

**17.** Zusammensetzung nach einem der vorhergehenden Ansprüche, dadurch gekennzeichnet, daß das kosmetisch akzeptable wässerige Medium Wasser oder ein Gemisch von Wasser und einem kosmetisch akzeptablen Lösungsmittel enthält.

**18.** Kosmetische Zusammensetzung in Form eines Schaums, dadurch gekennzeichnet, daß dieser Schaum durch Ausdehnung einer Zusammensetzung nach einem der vorhergehenden Ansprüche an der Luft entsteht.

**19.** Verfahren zur kosmetischen Behandlung keratinhaltiger Materialien wie der Haare, dadurch gekennzeichnet, daß es darin besteht, eine kosmetische Zusammensetzung wie vorstehend in einem der Ansprüche 1 bis 18 definiert auf die keratinhaltigen Materialien aufzutragen und gegebenenfalls anschließend, unter Umständen nach einer Einwirkzeit, mit Wasser auszuspülen.

**20.** Verwendung von assoziativen Polyurethanen, die mindestens eine hydrophile Sequenz, mindestens eine hydrophobe Sequenz und mindestens eine Urethaneinheit enthalten, zur Verbesserung der Eigenschaften von Schäumen, die durch Ausdehnung einer als Aerosol unter Druck stehenden Zusammensetzung auf der Basis eines anionischen Polymers und eines Treibmittels entstehen, wobei zumindest eines der beiden Polymeren ein Polymer mit Schaumbildungsvermögen ist.